# EUROPEAN PATENT SPECIFICATION

(11) **EP 1 235 524 B1**
(45) Date of publication and mention of the grant of the patent: **16.08.2006**
(21) Application number: 00972443.6
(22) Date of filing: 26.10.2000
(51) Int. Cl.: A61F 2/08, A61B 17/04, A61B 17/86

(54) **ORTHOPAEDIC LIGAMENT FIXATION SYSTEM**
FIXATIONSYSTEM FÜR EIN ORTHOPÄDISCHES BAND
SYSTEME ORTHOPEDIQUE DE FIXATION DE LIGAMENT

(30) Priority: 26.10.1999 AU PQ366099
(43) Date of publication of application: 04.09.2002
(73) Proprietor: Dr S. Fine & Dr G. Shar Pty. Ltd., Brisbane, Queensland 4120 (AU)
(72) Inventor: FINE, Stephen, Priv. Practice Rooms, Brisbane, Queensland 4120 (AU); MALEY, Kevin, John, Bundall, Queensland 4217 (AU); PEARCY, Mark, John, Capalaba, Queensland 4157 (AU)
(74) Representative: Stevens, Jason Paul
(86) International application number: PCT/AU2000/001312
(87) International publication number: WO 2001/030253

(56) References cited:
- EP-A2- 0 913 131
- WO-A-98/38938
- FR-A- 2 691 626
- FR-A- 2 725 615
- US-A- 4 013 071
- US-A- 5 152 790
- US-A- 5 425 733
- US-A- 5 643 321
- US-A- 5 690 676
- US-A- 5 957 953
- US-A- 6 041 485

## Description

THIS INVENTION relates to the field of orthopaedic anchors and in particular anchors for ligament or tendon graft fixation within bone tunnels.

Damaged and ruptured ligaments are relatively common occurrences as a result of traumatic incidents such as sporting injury and vehicle and workplace accidents. The most definitive and successful approach to these injuries involve surgical reconstruction of ligaments. One class of repairs involves surgical reconstruction of ligaments by replacing them with tendon grafts. Commonly, the ends of such grafts are secured within tunnels created in the bones of a patient Initially, the stability of the reconstructed ligament is reliant upon the mechanical fixation of the tendon graft to the host bone. Eventually, biological integration occurs between the graft and the host bone.

An advance which has provided particular advantages in post surgical results involves modern rehabilitation techniques based on early mobilisation of affected joints. It is essential for this early rehabilitative intervention that the mechanical fixation of the reconstructed ligaments be strong enough to withstand the forces generated on the tendon graft by early mobilisation or the fixation will fail. Such a failure often results in permanent damage and prejudice to the chances of a successful outcome from surgery. Various methods of mechanical fixation have been used in orthopaedic surgery to provide initial mechanical fixation strength which is adequate for the tendon graft and host bone to be held in firm contact until the graft is biologically incorporated into the host bone. No technique however has proved completely satisfactory.

US5957953 describes an adjustably expandable suture anchor comprising an outer member, and an inner member which is progressively inserted into the outer member until a desired expansion is reached.

FR2691626 describes a self tapping orthopaedic expansion screw for anchoring articulated prosthetic elements in a bone.

US4013071 describes a fastener comprising an external threaded fastening member and an internal spreading member.

FR2725615 describes an orthopaedic screw device comprising an inner screw member and an outer expansion member. When the screw is screwed in to the expansion member, blades in the outer surface of the expansion member anchor the device into the bone.

It is an object of the current invention to provide a bone fixation system which may be effectively used for ligament fixation. It is a further object to provide a surgeon with a useful choice of ligament fixation devices.

The present invention provides a ligament fixation system comprising :
a screw member, said screw member comprising a shank having:
   a proximate end and a remote end;
   a threaded external surface;
   at least one longitudinal slit; and
   a threaded longitudinal bore; and
   an expansion member, said expansion member comprising a threaded shaft for engagement of the threaded longitudinal bore;
   wherein insertion of the expansion member into the threaded longitudinal bore causes radial expansion of the screw member, characterised by:
   the threaded external surface having threads with an atraumatic profile, wherein the ridges of the threads are rounded thereby decreasing the tendency of the threads to cut.

Preferably, the shank has a head at its proximate end. The head may have at least one deformation for engaging an insertional tool.

The shank may be uniform in diameter. Alternatively, the shank may be longitudinally convergent in a direction away from the proximate end. Further alternatively, the shank may be longitudinally convergent in a direction toward the proximate end. As a further alternative, the shank may be regionally circumferentially expanded intermediate its two ends.

The at least one longitudinal slit may transect the head. Alternatively, the at least one slit may commence at an end of the shank remote from the head. The screw member may have at least two longitudinal slits.

The threaded longitudinal bore may have a constant diameter throughout its length. Alternatively, the threaded longitudinal bore may diverge circumferentially in an axial direction away from the proximate end. Further alternatively, the longitudinal bore may converge circumferentially in an axial direction away from the proximate end. The longitudinal bore may be a blind hole. Preferably, the longitudinal bore is a through hole.

The expansion member may have a proximal and distal end. The proximal end and adjacent region may be conically expanded in a direction towards the proximal end to thereby provide increasing radial expansion of the screw member during insertion of the expansion member. Alternatively, the distal end and adjacent region of the expansion member may be conically expanded to provide increasing radial expansion of the end of the screw member remote from the head during insertion of the expansion member. Further alternatively, the expansion member may have at least one circumferentially expanded region intermediate the proximal and distal end for regionally increasing the radial expansion of the screw member.

The expansion member preferably includes engagement means for engaging an insertional tool. The engagement means may be a slit for receiving a screw driver. Alternatively, the engagement means is a hexagonal recess.

Preferably, the screw member and expansion member are both cannulated for passage along a guide wire.

Preferably, the ligament fixation system further comprises:
an insertional tool, said insertional tool having an outer sleeve with an end adapted to engage the head of the screw member and an inner rotatable member adapted to engage the expansion member.

The outer sleeve may be in the form of a cylinder with an end adapted to engage a recess in the screw member by at least one interlocking projection. Alternatively or additionally, the end may have a recess to receive a corresponding projection of the screw member.

The inner rotatable member may be a screw driver with an end adapted to engage a receiving recess of the expansion member. The insertional tool may be cannulated for passage along a guide wire.

Preferably, the insertional tool indudes a torque gauge for determining the amount of torque applied to an expansion member.
FIG. 1 is a side view of a ligament fixation system of the present invention.
FIG. 2 is a longitudinal sectional view of the ligament fixation system of FIG. 1.
FIG. is a sectional view of a second embodiment of the ligament fixation system.
FIG. 4 is a sectional view of a third embodiment of the ligament fixation system.
FIG. 5 is a sectional view of a fourth embodiment of the ligament fixation system.
FIG. 6 is a sectional view of the embodiment of FIG. 5 when assembled.
FIG. 7 is a sectional view of a fifth embodiment of the ligament fixation system.
FIG. 8 is a sectional view of the embodiment of FIG. 7 when assembled.
FIG. 9 is a top view of an assembled screw member and expansion membe of the ligament fixation system.
FIG. 10 is a side part sectional view of a ligament fixation system, including an insertional tool.
FIG. 11 shows an embodiment of the ligament fixation system in operative position.

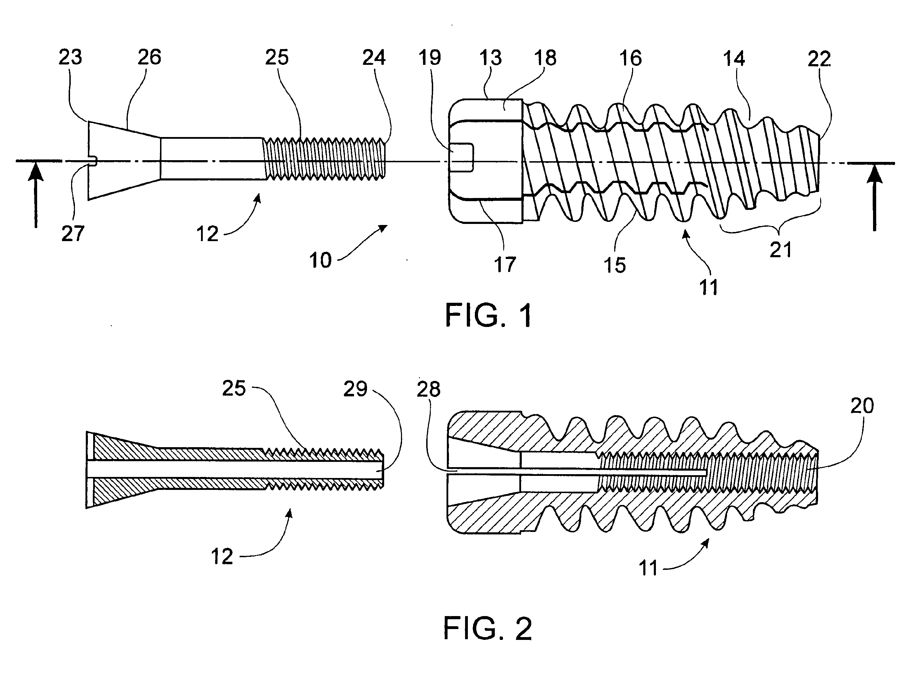

In FIG. 1 there is shown a ligament fixation system 10, comprising a screw member 11 and expansion member 12.

The screw member 11 has a head 13 at a proximate end, a shank 14 and a remote end 22.

Although a head is shown in all the figures, it is clear a screw member may be provided which has no head and a threaded external surface along its full length.

The shank 14 has a threaded external surface 15 in which the profile of the thread 16 is of an atraumatic form, in that the ridges of the threads are rounded thereby decreasing the tendency of the threads to cut. This is of considerable advantage when used with soft tissue grafts such as tendon grafts and ligaments, as the atraumatic profile 16 does not cut into the tissue and thereby shred it during installation. In addition, a shearing effect when in position is minimised. The screw member 11 has two longitudinal slits 17,18 visible. Both slits 17, 18 transect or traverse the head 13. A third longitudinal slit is not visible in this view. The head 13 has a surface deformation in the form of recess 19 for receiving a matching projection on an insertional tool.

The screw member 11 has a threaded longitudinal bore 20 which is not visible in this view, but is apparent in FIG. 2. The thread preferably runs along a majority of the bore. The shank 14 in this embodiment has converging or narrowing circumferential threads 21 which converge in a direction away from head 13 towards the remote end 22. This narrowing of the shank 14 provides an improved ability to screw the screw member 11 into position.

The expansion member 12 has a proximal end 23, a distal end 24 and a shaft 25. The shaft 25 has a conically expanded region 26 adjacent to the proximal end 23 and a threaded section. The proximal end 23 has engagement means for receiving an insertional tool in the form of a recess 27 which may receive a screw driver head (not shown).

FIG. 2 is a sectional view showing the positioning of the screw member 11 and expansion member 12 with the threaded shaft 25 aligned for entry into the threaded bore 20. In use, a tendon graft is surgically prepared. In this specification, the expression tendon graft is intended to include all forms of tissue and, indeed, artificial implants used in ligament repair. It may also include ligamentous tissue itself. Likewise, the term ligament may include a tendon graft or other form of ligament repair. The graft may have a bone block at its end, although this is not essential. The invention is particularly well adapted to anchor a graft comprising only soft tissue. The end of the tendon graft is located in a prepared bone tunnel. Screw member 11 is then positioned by screwing into the gap between one side of the end of the graft and the tunnel wall. It is carefully positioned and the atraumatic profile threads 16 gently engage an edge of the tendon graft. Once the screw member 11 is satisfactorily placed, the expansion member 12 is screwed into the threaded longitudinal bore 20. This may be accomplished by using an insertional tool which will be described below. The head 13 may be stabilised while the expansion member 12 is screwed into position. As the expansion member 12 is advanced the conically expanded end 26 flares the head 13 and proximal region of the shank 14 by increasing the spacing of the slits 17, 18, 28, thereby causing a circumferential increase in a region of the screw member 11 and increasing the pressure exerted on the tendon graft to urge it into closer and pressurised contact with the bone tunnel wall. This serves the dual purpose of increasing the stability and strength of the anchoring of the graft as well as enhancing the union between the graft and the patient's host bone. The expansion member 12 may be tightened to a preselected level by using a torque gauge on an insertional tool. A third slit 28 is apparent in FIG. 2. Although three slits have been described in this embodiment, it is clear a greater or lesser number of slits may be used as appropriate for different indications. Also visible in FIG. 2 is a cannula 29 in the form of a through hole in the expansion member 12. This provides the capacity to use a guide wire in endoscopic installation of the screw member 11 and expansion member 12. The guide wire may be passed through the threaded bore 20 and cannula 29 to provide the directional control necessary for such a procedure.

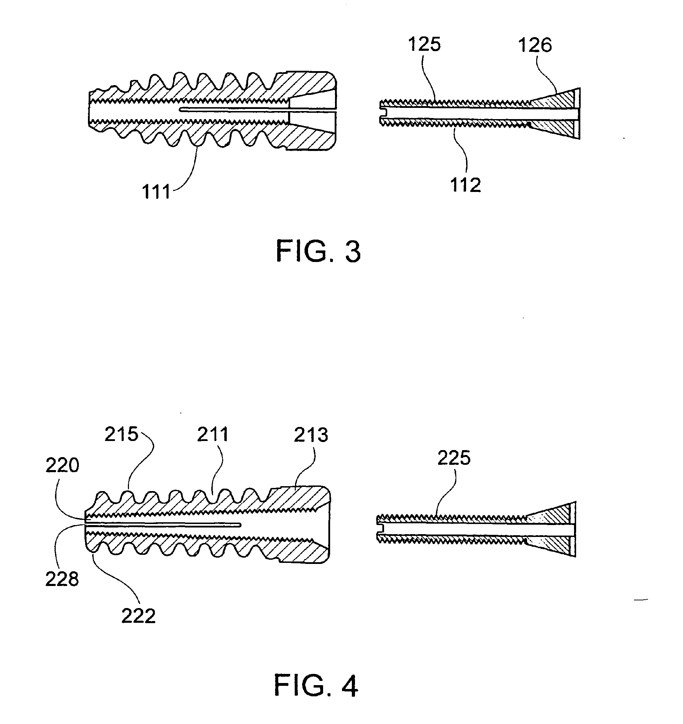

FIG. 3 shows a second embodiment of a ligament fixation system which is substantially similar to the first embodiment except that the threaded shaft 125 is threaded for its entire length up to the conically expanded region 126. This arrangement provides additional drive capacity on full insertion of the expansion member 112 into the screw member 111.

In FIG. 4 a third embodiment is shown which is substantially similar to that shown in FIG. 3, except the threaded longitudinal bore 220 narrows or converges in a direction away from head 213. As the shaft 225 is inserted it will expand the remote end 222 and adjacent shank area of the screw member 211 so that the external threaded surface 215 ends up with threads which are substantially parallel or which may in fact be flared beyond parallel in a further embodiment. In this embodiment the slits commence at the remote end 220 as shown by slit 228. In certain applications it may be appropriate to have a number of slits, some of which commence at the head 213 and others at the remote end 220.

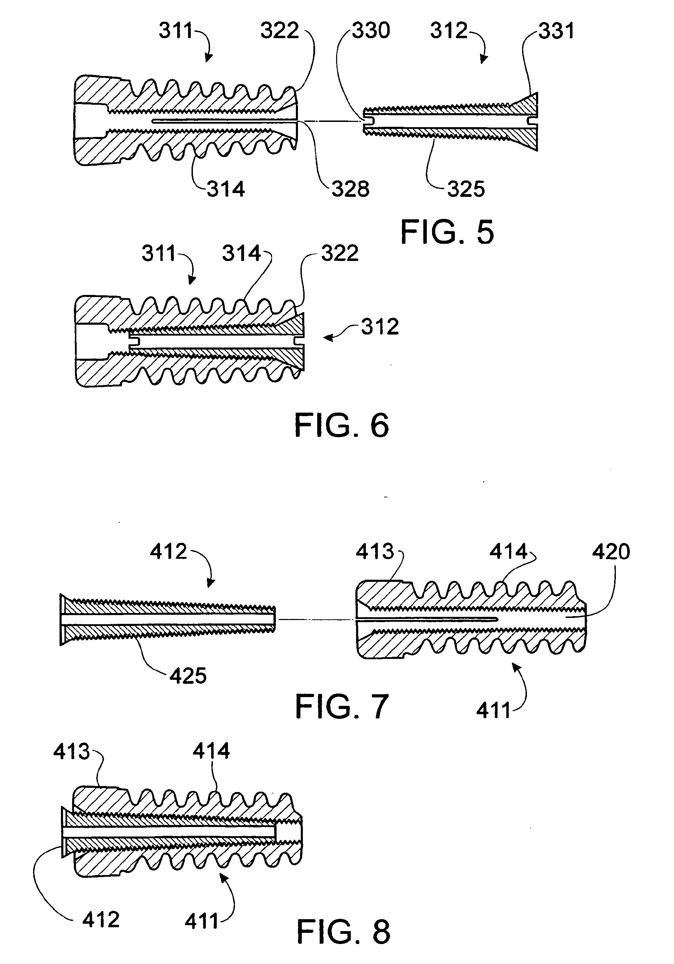

FIG. 5 discloses an embodiment in which the expansion member 312 inserts from the remote end 322 of the screw member 311. Again slit 328 commences at remote end 322. The expansion member 312 has a slot 330 for receiving the end of a tool such as a screwdriver during installation. In this embodiment the threaded shaft 325 converges in a direction away from expansion member head 331 so that installation of the expansion member 312 will lead to an increased radial expansion of the remote end 322 compared with that of the rest of the shank 314.

FIG. 6 shows the components of FIG. 5 combined. The end 322 has been expanded, while the threads of both components are engaged and the expansion member 312 is advanced in a retrograde fashion into the screw member 311. This arrangement may provide increased localised compressive force if required at the remote end 322. Alternatively, the arrangement may be such as to provide a constant diameter of the expanded shank 314 when in position.

FIG. 7 shows separated components of a fourth embodiment of the ligament fixation system in which the expansion member 412 has a converging threaded shaft 425 for insertion in threaded bore 420. As the expansion member 412 is advanced it provides increasing radial expansion of the head 413 and proximal region of the shank 414.

This is highlighted in FIG. 8 which shows the profile of the screw member 411 seen in FIG. 7 which had substantially parallel outer limits and which are now flaring at the head 413 end of shank 414.

In one embodiment, the slit is a single slit which runs the whole length of the screw member.

The screw member and/or the expansion member may be made from metal, plastic or other suitable material.

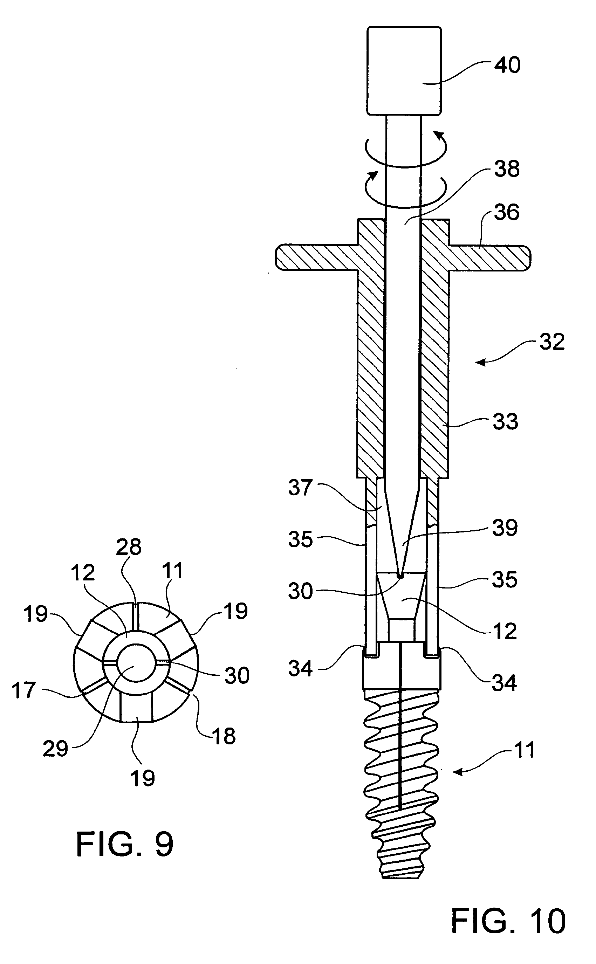

FIG. 9 shows a top view of screw member 11 with expansion member 12 inserted. A surface deformation in the form of recesses 19 is shown for receiving an end of an insertional tool. Slits 17, 18, 28 are also apparent. The expansion member 12 has a slot 30 for receiving the end of a screwdriver and cannula 29 is also apparent.

FIG. 10 shows the embodiment of FIG. 9 engaged with insertional tool 32. The expansion member 12 is partially inserted into the screw member 11. The insertional tool 32 has an outer sleeve 33 (shown in section) with an end 34 adapted to insert into the recesses 19 of FIG. 9 in this case in the form of extensor prongs. The outer sleeve 33 has a handle 36 to facilitate manipulation of the sleeve 33 and screwing insertion of screw member 11. The outer sleeve 33 has an internal bore 37 with a rotatable member 38 having a screwdriver head 39 for insertion in slot 30 of expansion member 12.

In use, the screw member 11 is located within a bone tunnel and alongside a tendon graft by rotation of outer sleeve 33. The screw member 11 may then be stabilised using outer sleeve 33 while the expansion member 12 is installed by rotation of rotatable member 38. It is important to maintain the screw member 11 in static position as it would otherwise tend to advance in the bone tunnel and change position relative to the tendon graft. The insertional tool may include a torque gauge 40 (shown schematically) which allows a surgeon to apply a preselected level of torque to expansion member 12 relative to the screw member 11 and thereby provide expansion of the screw member 11 which is suitable for the particular situation and which is neither too little, which may result in failure of anchoring, nor too large, which may result in damage of the compressed tissue.

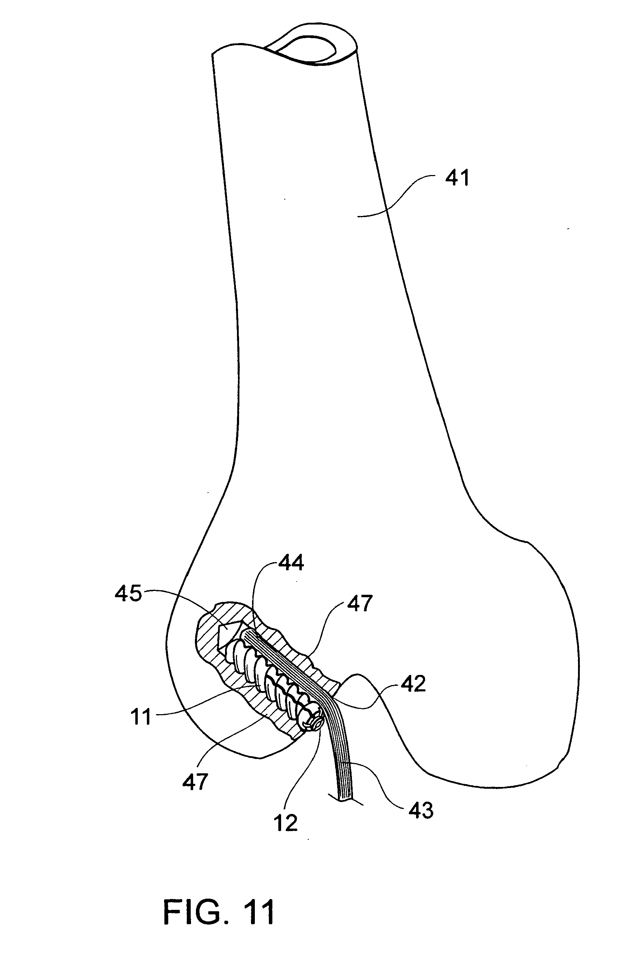

FIG. 11 shows an embodiment of the ligament fixation system in use during an anterior-cruciate ligament repair. Although this surgical procedure is described, it is clear to a skilled addressee that the application of the ligament fixation system is not restricted to this procedure alone. Nor, in fact, is it restricted to anchoring of tendon grafts alone. It is possible that the system may find application in other orthopaedic procedures and the description is not intended to limit those applications or the extent of the patent sought in any way.

A tunnel is drilled in the distal femur of a patient at the attachment site 42 of the anterior-cruciate ligament. A complementary tunnel is created in the tibia (not shown). The procedure may be performed endoscopically or via an open surgical approach. A tendon graft 43 is harvested from an appropriate anatomical source such as the hamstring tendons or patella tendon.

An end 44 of the tendon graft 43 is pulled into the bone tunnel 45 . If performed endoscopically a guide wire may be passed alongside the tendon graft 43 into the bone tunnel 45 and a screw member 11 is passed along the guide wire as is an insertional tool (not shown). The screw member 11 is turned so that its atraumatic threads engage the wall of the bone tunnel 45 as well as the end 44 of the tendon graft 43 leading to the advancement of the screw member 11 into the bone tunnel 45. Once the screw member 11 is placed to the satisfaction of the surgeon fully within the tunnel, an expansion member 12 is also advanced along the wire. The outer sleeve of the insertional screw stabilises the screw member 11 while the expansion screw 12 is rotated into firm and expanding contact with the screw member 11. The expansion member 12 may have a conical shape which expands the outer screw, thereby improving the fixation of the tendon graft within the tunnel. A similar procedure is then used to place the device and graft in a tibial bone tunnel securing the tendon graft at each of its two ends.

This view also shows the end 44 of tendon graft 43 conforming to the external threaded surface of screw member 11 and urged into close proximity with wall 47 of the bone tunnel 45. Similarly, the external threaded surface has formed a matching thread in wall 47 of the bone tunnel 45 as the expansion member 12 is fully inserted. An alternative method may involve screwing the expansion member into the screw member from a remote end of the screw member.

Throughout the specification, the aim has been to describe the preferred embodiments of the invention without limiting the invention to any one embodiment or specific collection of features. Various changes and modifications may be made to the embodiments described and illustrated without departing from the scope of present invention as defined by the appended claims.

## Claims

1. A ligament fixation system (10) comprising:
a screw member (11), said screw member comprising a shank (14) having:
a proximate end and a remote end (22); a threaded external surface (15); at least one longitudinal slit (17); and a threaded longitudinal bore (20); and an expansion member (12), said expansion member comprising a threaded shaft for engagement of the threaded longitudinal bore;
wherein insertion of the expansion member into the threaded longitudinal bore causes radial expansion of the screw member **characterised by**:
the threaded external surface having threads with an atraumatic profile (16), wherein the ridges of the threads are rounded thereby decreasing the tendency of the threads to cut.

2. The ligament fixation system of claim 1, wherein the shank
further comprises a head (13) at the proximate end.

3. The ligament fixation system of claim 2, wherein the head (13)
has at least one surface deformation (19) for engaging an insertion a I tool.

4. The ligament fixation system of claim 1, wherein the shank is of
a constant diameter prior to insertion of the expansion member.

5. The ligament fixation system of claim 1, wherein the shank is
longitudinally convergent in the direction towards the remote end prior to insertion of the expansion member.

6. The ligament fixation system of claim 1, wherein the shank is
longitudinally convergent in the direction toward the proximate end prior to
insertion of the expansion member.

7. The ligament fixation system of claim 1, wherein the shank is regionally circumferentially expanded intermediate the proximate and remote
ends.

8. The ligament fixation system of claim 1, wherein the shank ha three longitudinal slits.

9. The ligament fixation system of claim 2, wherein the at least one longitudinal slit transects the head.

10. The ligament fixation system of claim 1, wherein the at least
one longitudinal slit commences at the remote end of the shank

11. The ligament fixation system of claim 1, wherein the threaded
longitudinal bore has a constant diameter throughout its length.

12. The ligament fixation system of claim 1, wherein the threaded
longitudinal bore diverges circumferentially in a direction away from the
proximate end.

13. The ligament fixation system of claim 1, wherein the threaded
longitudinal bore nverges circumferentially in a direction away from the
proximate end.

14. The ligament fixation system of claim 1, wherein the threaded
longitudinal bore is a blind hole.

15. The ligament fixation system of claim 1, wherein the threaded
longitudinal bore is a through hole.

16. The ligament fixation system of claim 1, wherein a proximal end
and adjacent region of the expansion member are conically expanded.

17. The ligament fixation system of claim 1, wherein a distal end
and adjacent region of the expansion member are conically expanded.

18. The ligament fixation system of claim 1, wherein the expansion
member has at least one circumferentially expanded region intermediate the
proximal and distal ends.

19. The ligament fixation system of claim 1, wherein the expansion
member includes engagement means for engaging an insertional tool.

20. The ligament fixation system of any one of the preceding
claims, wherein the screw member and expansion member are both
cannulated for passage along a guide wire.

21. The ligament fixation system of any one of the preceding
claims, further including an insertional tool.

22. The ligament fixation system of claim 21, wherein the
insertional tool comprises an outer sleeve with an end adapted to engage the
head of the screw member and an inner rotatable member adapted to
engage the expansion member.

23. The ligament fixation system of claim 22, wherein the outer
sleeve is a cylinder with an end having at least one projection for engaging a
recess in the screw member.

24. The ligament fixation system of claim 22, wherein the outer
sleeve is a cylinder with an end having at least one recess for receiving a
projection on the screw member.

25. The ligament fixation system of claim 22, wherein the inner
rotatable member is a screwdriver.

26. The ligament fixation system of any one of claims 22 to 25,
wherein the insertional tool is cannulated for passage along a guide wire.

## Patentansprüche

1. Bänder- bzw. Ligament-Befestigungssystem (10), das enthält:
ein Schraubenelement (11), das einen Schaft (14) aufweist, der enthält:
ein nahes Ende und ein fernes Ende (22); eine mit Gewinde versehene äußere Oberfläche (15); wenigstens einen longitudinalen Schlitz (17); und eine mit Gewinde versehene longitudinale Bohrung (20); und ein Ausdehnungselement (12), wobei das Ausdehnungselement einen mit einem Gewinde versehenen Schaft aufweist, der mit der mit Gewinde versehenen longitudinalen Bohrung in Eingriff bringbar ist;
wobei das Einführen des Ausdehnungselements in die mit Gewinde versehene longitudinale Bohrung eine radiale Ausdehnung des Schraubenelements bewirkt, **dadurch gekennzeichnet, dass**:
die mit Gewinde versehene äußere Oberfläche Gewinde mit einem atraumatischen Profil (16) aufweist, wobei die Stege der Gewinde abgerundet sind, wodurch die Schneidetendenz der Gewinde verringert wird.

2. Ligament-Befestigungssystem nach Anspruch 1, bei dem der Schaft ferner am nahen Ende einen Kopf (13) aufweist.

3. Ligament-Befestigungssystem nach Anspruch 2, bei dem der Kopf (13) wenigstens eine Oberflächenverformung (19) aufweist, die mit einem Einführungswerkzeug in Eingriff gelangen soll.

4. Ligament-Befestigungssystem nach Anspruch 1, bei dem der Schaft vor dem Einführen des Ausdehnungselements einen konstanten Durchmesser besitzt.

5. Ligament-Befestigungssystem nach Anspruch 1, bei dem der Schaft vor dem Einführen des Ausdehnungselements longitudinal in Richtung zu dem fernen Ende zusammenläuft bzw. konvergiert.

6. Ligament-Befestigungssystem nach Anspruch 1, bei dem der Schaft vor dem Einführen des Ausdehnungselements longitudinal in Richtung zu dem nahen Ende zusammen läuft.

7. Ligament-Befestigungssystem nach Anspruch 1, bei dem der Schaft zwischen dem nahen Ende und dem fernen Ende gebietsweise in Umfangsrichtung erweitert ist.

8. Ligament-Befestigungssystem nach Anspruch 1, bei dem der Schaft drei longitudinale Schlitze aufweist.

9. Ligament-Befestigungssystem nach Anspruch 2, bei dem der wenigstens eine longitudinale Schlitz den Kopf schneidet.

10. Ligament-Befestigungssystem nach Anspruch 1, bei dem der wenigstens eine longitudinale Schlitz an dem fernen Ende des Schafts beginnt.

11. Ligament-Befestigungssystem nach Anspruch 1, bei dem die mit Gewinde versehene longitudinale Bohrung über ihre gesamte Länge einen konstanten Durchmesser aufweist.

12. Ligament-Befestigungssystem nach Anspruch 1, bei dem die mit Gewinde versehene longitudinale Bohrung in Umfangsrichtung in einer von dem nahen Ende wegweisenden Richtung auseinander läuft.

13. Ligament-Befestigungssystem nach Anspruch 1, bei dem die mit Gewinde versehene longitudinale Bohrung in Umfangsrichtung in einer von dem nahen Ende wegweisenden Richtung zusammen läuft.

14. Ligament-Befestigungssystem nach Anspruch 1, bei dem die mit Gewinde versehene longitudinale Bohrung ein Blindloch ist.

15. Ligament-Befestigungssystem nach Anspruch 1, bei dem die mit Gewinde versehene longitudinale Bohrung ein Durchgangsloch ist.

16. Ligament-Befestigungssystem nach Anspruch 1, bei dem ein proximales Ende und ein angrenzender Bereich des Ausdehnungselements konisch erweitert sind.

17. Ligament-Befestigungssystem nach Anspruch 1, bei dem ein distales Ende und ein angrenzender Bereich des Ausdehnungselements konisch erweitert sind.

18. Ligament-Befestigungssystem nach Anspruch 1, bei dem das Ausdehnungselement zwischen dem proximalen Ende und dem distalen Ende wenigstens einen in Umfangsrichtung erweiterten Bereich aufweist.

19. Ligament-Befestigungssystem nach Anspruch 1, bei dem das Ausdehnungselement Eingriffmittel für den Eingriff eines Einführungswerkzeugs aufweist.

20. Ligament-Befestigungssystem nach einem der vorhergehenden Ansprüche, bei dem das Schraubenelement und das Ausdehnungselement jeweils mit einem Kanal versehen sind, um sich längs eines Führungsdrahts zu bewegen.

21. Ligament-Befestigungssystem nach einem der vorhergehenden Ansprüche, das ferner ein Einführungswerkzeug aufweist.

22. Ligament-Befestigungssystem nach Anspruch 21, bei dem das Einführungswerkzeug eine äußere Hülse mit einem Ende, das so beschaffen ist, dass es mit dem Kopf des Schraubenelements in Eingriff bringbar ist, und ein inneres drehbares Element, das so beschaffen ist, dass es mit dem Ausdehnungselement in Eingriff bringbar ist, aufweist.

23. Ligament-Befestigungssystem nach Anspruch 22, bei dem die äußere Hülse ein Zylinder ist, wovon ein Ende wenigstens einen Vorsprung aufweist, der mit einer Aussparung im Schraubenelement in Eingriff bringbar ist.

24. Ligament-Befestigungssystem nach Anspruch 22, bei dem die äußere Hülse ein Zylinder ist, wovon ein Ende wenigstens eine Aussparung für die Aufnahme eines Vorsprungs am Schraubenelement aufweist.

25. Ligament-Befestigungssystem nach Anspruch 22, bei dem das innere drehbare Element ein Schraubendreher bzw. -zieher ist.

26. Ligament-Befestigungssystem nach einem der Ansprüche 22 bis 25, bei dem das Einführungswerkzeug mit einem Kanal versehen bzw. kanülisiert ist, um sich längs eines Führungsdrahts zu bewegen.

## Revendications

1. Système de fixation de ligament (10) comprenant :
un élément de vis (11), ledit élément de vis comprenant une tige (14) ayant :
une extrémité proximale et une extrémité distante (22) ; une surface externe filetée (15) ; au moins une fente longitudinale (17) ; un alésage longitudinal fileté (20) ; et un élément de dilatation (12), ledit élément de dilatation comprenant une tige filetée pour la mise en prise de l'alésage longitudinal fileté ;
dans lequel l'insertion de l'élément de dilatation dans l'alésage longitudinal fileté provoque la dilatation radiale de l'élément de vis **caractérisé par** :
la surface externe filetée qui a des filetages avec un profil atraumatique (16), dans lequel les parties saillantes des filetages sont arrondies, diminuant ainsi la tendance des filetages à couper.

2. Système de fixation de ligament selon la revendication 1, dans lequel la tige comprend en outre une tête (13) au niveau de l'extrémité proximale.

3. Système de fixation de ligament selon la revendication 2, dans lequel la tête (13) a au moins une déformation de surface (19) pour mettre en prise un outil d'insertion.

4. Système de fixation de ligament selon la revendication 1, dans lequel la tige a un diamètre constant avant l'insertion de l'élément de dilatation.

5. Système de fixation de ligament selon la revendication 1, dans lequel la tige est longitudinalement convergente dans la direction allant vers l'extrémité distante avant l'insertion de l'élément de dilatation.

6. Système de fixation de ligament selon la revendication 1, dans lequel la tige est longitudinalement convergente dans la direction allant vers l'extrémité proximale avant l'insertion de l'élément de dilatation.

7. Système de fixation de ligament selon la revendication 1, dans lequel la tige est dilatée de manière circonférentielle par région entre les extrémités proximales et distantes.

8. Système de fixation de ligament selon la revendication 1, dans lequel la tige a trois fentes longitudinales.

9. Système de fixation de ligament selon la revendication 2, dans lequel la au moins une fente longitudinale coupe transversalement la tête.

10. Système de fixation de ligament selon la revendication 1, dans lequel la au moins une fente longitudinale commence au niveau de l'extrémité distante de la tige.

11. Système de fixation de ligament selon la revendication 1, dans lequel l'alésage longitudinal fileté a un diamètre constant sur toute sa longueur.

12. Système de fixation de ligament selon la revendication 1, dans lequel l'alésage longitudinal fileté diverge de manière circonférentielle dans une direction à distance de l'extrémité proximale.

13. Système de fixation de ligament selon la revendication 1, dans lequel l'alésage longitudinal fileté converge de manière circonférentielle dans une direction à distance de l'extrémité proximale.

14. Système de fixation de ligament selon la revendication 1, dans lequel l'alésage longitudinal fileté est un trou borgne.

15. Système de fixation de ligament selon la revendication 1, dans lequel l'alésage longitudinal fileté est un trou de passage.

16. Système de fixation de ligament selon la revendication 1, dans lequel une extrémité proximale et une région adjacente de l'élément de dilatation sont dilatées de manière conique.

17. Système de fixation de ligament selon la revendication 1, dans lequel une extrémité distale et une région adjacente de l'élément de dilatation sont dilatées de manière conique.

18. Système de fixation de ligament selon la revendication 1, dans lequel l'élément de dilatation a au moins une région dilatée de manière circonférentielle entre les extrémités proximales et distales.

19. Système de fixation de ligament selon la revendication 1, dans lequel l'élément de dilatation comprend des moyens de mise en prise pour mettre en prise un outil d'insertion.

20. Système de fixation de ligament selon l'une quelconque des revendications précédentes, dans lequel l'élément de vis et l'élément de dilatation sont tous les deux rainurés pour le passage le long d'un fil guide.

21. Système de fixation de ligament selon l'une quelconque des revendications précédentes, comprenant en outre un outil d'insertion.

22. Système de fixation de ligament selon la revendication 21, dans lequel l'outil d'insertion comprend un manchon externe avec une extrémité adaptée pour mettre en prise la tête de l'élément de vis et un élément rotatif interne adapté pour mettre en prise l'élément de dilatation.

23. Système de fixation de ligament selon la revendication 22, dans lequel le manchon externe est un cylindre avec une extrémité ayant au moins une saillie pour mettre en prise un renfoncement dans l'élément de vis.

24. Système de fixation de ligament selon la revendication 22, dans lequel le manchon externe est un cylindre avec une extrémité ayant au moins un renfoncement pour recevoir une saillie sur l'élément de vis.

25. Système dé fixation de ligament selon la revendication 22, dans lequel l'élément rotatif interne est un tournevis.

26. Système de fixation de ligament selon l'une quelconque des revendications 22 à 25, dans lequel l'outil d'insertion est rainuré pour le passage le long d'un fil guide.
